# EUROPÄISCHE PATENTSCHRIFT

(11) **EP 1 327 412 B1**
(45) Veröffentlichungstag und Bekanntmachung des Hinweises auf die Patenterteilung: **15.08.2007**
(21) Anmeldenummer: 03000284.4
(22) Anmeldetag: 09.01.2003
(51) Int. Cl.: A61B 3/135, A61B 3/117

(54) **Anordnung zur Beleuchtung der Linse eines menschlichen Auges**
Apparatus for illuminating the lens of a human eye
Dispositif pour illuminer la lentille de l'oeil humain

(30) Priorität: 10.01.2002 DE 10200718
(43) Veröffentlichungstag der Anmeldung: 16.07.2003
(73) Patentinhaber: Carl Zeiss Meditec AG, 07745 Jena (DE)
(72) Erfinder: Wrobel, Walter, 07743 Jena (DE); Koschmieder, Ingo, 07743 Jena (DE); Donnerhacke, Karl-Heinz, 07747 Jena (DE)
(74) Vertreter: Geyer, Fehners & Partner

(56) Entgegenhaltungen:
- WO-A-01/71411
- WO-A-02/26121
- DE-A- 19 812 050
- DE-A- 19 943 735
- US-A- 5 202 708
- US-A- 5 784 146
- US-B1- 6 275 718

## Beschreibung

Die vorliegende Erfindung betrifft eine Anordnung zur Erzeugung einer variablen Beleuchtung bzw. Bestrahlung für die Diagnose und Therapie, insbesondere am menschlichen Auge, sowie ein Verfahren zu dessen Anwendung. Das von der variablen Beleuchtung beleuchtete Objekt kann dabei sowohl ein künstliches Objekt als auch ein biologisches Gewebe sein. Bei einem Auge ist die Bestrahlung der Augenlinse aber auch anderer Augenabschnitte wie Kornea, Retina oder Fundus möglich. Insbesondere kann die Erfindung für die Feinabstimmung von in das Auge eingebrachten photosensitiven Kunststoffen (gemäß WO 00/41650 und WO 01/71411) eingesetzt werden. Bei dieser Art von Kunststoffen werden durch Bestrahlung Polymerisationsvorgänge angeregt, die irreversible chemische Veränderungen der Substanz zur Folge haben. Durch diese Vorgänge können Brechungsindex, geometrische Form und/oder Transmissionsverhalten für die sichtbare Nutzstrahlung bzw. die geometrische Form des Kunststoffkörpers definiert verändert werden. Dadurch kann ein fehlerreduziertes Sehen ermöglicht werden.

In den Patentschriften WO 00/41650 und WO 01/71411 werden Linsen, insbesondere Intraokularlinsen (IOL) beschrieben, bei denen durch Bestrahlung die Polymerisation einer in der Linse enthaltenen Polymermatrix angeregt und dadurch der Brechungsindex oder die Form der Gesamtlinse verändert werden kann. Bei implantierten IOL besteht das Problem, dass bei ca. der Hälfte der Patienten eine akzeptable Sehleistung nur durch zusätzliche Korrektionsmittel wie Brillen oder Kontaktlinsen erreicht werden kann. Dies resultiert u.a. aus Fehlern bei der Augenvermessung, Abweichungen bei der Positionierung der IOL und/oder durch den Wundheilungsprozess. Mit den beschriebenen IOL wird durch eine gezielte Bestrahlung eine Korrektur der bereits implantierten IOL ermöglicht, indem durch Änderung des Brechungsindex, der Transmissionseigenschaften oder Änderungen der optisch wirksamen Form eine Anpassung an die tatsächlichen Gegebenheiten erfolgt. Die Bestrahlung der IOL zur Anregung des Polymerisationsvorganges erfolgt vorzugsweise mittels Laserquellen oder Lampen, die einen hohen UV-Anteil des Lichtes aussenden. Hierbei dient als Bestrahlungsquelle ein He/Cd-Laser bzw. eine Xe/Hg-Lampe. Die eventuell erforderlichen Beleuchtungsstrukturen werden in der Regel mit Hilfe mechanischer Blenden und/oder Filtern erzeugt.

Derartige Anordnungen haben jedoch die Nachteile, dass der Mustervorrat durch feste Blenden begrenzt ist, dass keine Intensitätsverteilung innerhalb der Blendenmuster möglich ist und dass dynamische Vorgänge höchstens durch manuelle Umschaltung und somit kaum realisierbar sind. Die erzeugten Beleuchtungsmuster sind zudem nicht an individuelle Befunddaten anpassbar, nicht adaptiv und nicht für eine online-Regelung geeignet.

Die DE 199 43 735 A1 beschreibt ein Verfahren und eine Vorrichtung zur gezielten Bestrahlung eines Auges mittels Licht aus dem UV-A und/oder dem sichtbaren nahinfraroten Wellenlängenbereich. Durch die Bestrahlung werden irreversible chemische Veränderungen der Augenlinsen-Substanz hervorgerufen, die eine Veränderung des Brechungsindex und/oder der Transmissionseigenschaften für die sichtbare Nutzstrahlung zur Folge haben und dadurch ein fehlerreduziertes Sehen ermöglichen. Die erfolgreiche Behandlung setzt dabei eine möglichst engmaschige und vollflächige Bestimmung der Verteilung der Brechkraft des zu behandelnden Auges voraus. Aus diesen Werten werden die nach der Behandlung gewünschte Brechkraftverteilung und die dafür erforderlichen Daten der Bestrahlung ermittelt. Für die Dauer der Behandlung ist es jedoch meist unerlässlich eine Fixierung des Augapfels vorzunehmen.

In der nachveröffentlichten WO 02/26121 und in WO 02/31576 werden Lösungen für die Bestrahlung optischer Linsen oder Linsensysteme aus photosensitiven Kunststoffen (gemäß WO 00/41650 und WO 01/71411) beschrieben, die als Intraokularlinsen bereits in das Auge implantiert sind. Bei dieser Lösung werden die betreffenden Bestrahlungsmuster anhand einer zuvor durchgeführten Wellenfront-Analyse durch ein Computerprogramm bestimmt. Neben einem Diagnoseelement zur Kontrolle vor, während und nach der Bestrahlung ist ein chirurgisches Mikroskop zur zusätzlichen visuellen Beobachtung vorhanden. Nachteilig bei dieser Lösung wirkt sich jedoch aus, dass nur ein Fixierlicht für den Patienten vorhanden ist. Erfahrungsgemäß fällt es den Patienten schwer sich für die Dauer der Behandlung auf ein festes Fixierlicht zu konzentrieren, so dass es trotzdem zu Bewegungen des Auges kommen kann.

In der DE 198 12 050 A1 sind ein Verfahren und eine Anordnung zur Beleuchtung bei einem Augenmikroskop beschrieben. Die verschiedensten Leuchtmarkengeometrien werden mit Hilfe opto-elektronischer Bauelemente erzeugt und auf den Augenvorder- oder Hintergrund projiziert. Diese Lösung dient der allgemeinen Untersuchung des Auges. Eine Anordnung zur Erzeugung von Schnittbildern in transparenten Medien ist in der DE 101 55 464 vorgesehen. Ebenfalls existiert ein ophthalmologisches Untersuchungsgerät mit dem neben einer allgemeinen Augenuntersuchung auch eine perimetrische Untersuchung ermöglicht wird (DE 101 51 314). Die Lösungen dieser beiden Schriften sehen ebenfalls die Verwendung opto-elektronischer Bauelemente zur Erzeugung der Beleuchtungsmarken und - muster vor.

In US-B1-6 275 718 sind ein Verfahren und eine entsprechende Vorrichtung beschrieben, die zur in-vivo Abbildung von Cornea-Gewebe dient und im allgemeinen einen Laserstrahl umfaßt, der eine im wesentlichen planare Konfiguration hat, um einen Querschnittsbereich eines Auges eines Patienten zu beleuchten und den Laserstrahl durch Moelküle in dem Cornea-Gewebe zu streuen. Das gestreute .Laserlicht wird detektiert, um ein Querschnittsbild des Cornea-Gewebes zu bilden. Daten über die Corneadicke und -topographie können erzeugt werden, um von Augenchirurgen bei der Planung des genauen Krümmungsprofils benutzt zu werden, das photoablativ im Stroma-Gewebe des jeweiligen Patienten gebildet wird, um eine gewünschten optischen Korrekturgrad in seinem oder ihrem Auge zu erreichen. Zusätzlich wird eine miniaturisierte Vorrichtung zur Benutzung in dem Bereich beschrieben, die ein Gehäuse mit einer Laserquelle zur Beleuchtung des Patientenauges und eine Kamera zur Aufzeichnung des reflektierten Lichts und entweder ein gesteuertes bewegliches Ziel für das untersuchte Patientenauge, auf das fokussiert werden soll, oder eine Einrichtung zum Scannen des Strahls umfaßt.

In US-A- 5 784 146 ist eine ophtalmologische Meßvorrichtung beschrieben, die optische Systeme zur Fokussierung und Projektion eines Laserstrahls zur Benutzung bei einer Messung auf einem vorderen Bereich eines zu untersuchenden Auges und zum Empfangen von von dem inneren Gewebe des vorderen Bereichs des Auges gestreutem Licht durch Führung des gestreuten Lichts auf ein photoelektrisches Element, und zur Messung von Geweben und Komponenten in den inneren Teilen des vorderen Bereichs des Auages auf der Basis von von dem photoelektrischen Element übertragenen Ausgangssignalen. Eine Ausrichtung wird durchgeführt, indem eine in eime Lichtweg in dem Laserstahlprojektionssystem angeorddnete Zylinderlinse zur Bildung eines Laserstrahls mit einem schlitzförmigen Querschnitt angenähert wird, wobei ein Bild eines eine Abschnitt des des vorderen Bereichs des Auges, der durch den Laserstrahl beleuchtet wird, beobachtet wird, oder indem ein Laserstrahl von einer Laserstrahlablenkeinrichtung abgelenkt wird, wobei ein Bild eines Abschnitts des vorderen Bereichs des Auges beobachtet wird, der von dem Laserstrahl durch ein optisches Beobachtungssystem bereichsweise beleuchtet wird.

In US-A-5 202 708 ist eine Vorrichtung zum Photographieren eines Bildes einer Kristalllinse bei rückwärtiger Beleuchtung beschrieben, in der ein zu untersuchendes Auge durch Beleuchtungslicht projiziert und die Kristalllinse rückwärtig durch an dem Fundus reflektiertes Licht beleuchtet wird. Die Vorrichtung umfaßt: eine Ausrichteinrichtung, die ein optisches Photographiesystem der Vorrichtung auf einen vorgegebenen Bereich eines zu untersuchenden Auges ausrichtet, eine Speichereinrichtung, die die Position des von Ausrichteinrichtung ausgerichteten optischen Photographiersystems speichert, eine Ursprungsberechnungseinrichtung, die einen bestimmten Bereich des zu untersuchenden Auges als Ursprung einer Bildaufnehmerposition des optischen Photographiersystems auf der Basis der Positionsinformatione der Speichereinrichtung berechnet, ein Detektionsmittel, das eine Bewegungsgröße der Bildaufnehmerposition des optischen Photographiersystems detektiert, und eine Anzeigeeinrichtung, die die die Bildaufnahmerposition des optischen Photographiersystems auf der Basis des Detektionsergebnisses der Detektionseinrichtung anzeigt.

Der vorliegenden Erfindung liegt die Aufgabe zugrunde, eine Anordnung zur Beleuchtung/Bestrahlung eines menschlichen Auges zu Therapiezwecken sowie zur Korrektur der Eigenschaften bereits im Auge implantierter photosensitiver Kunststoffe zu entwickeln. Durch diese Korrektur soll eine optimierte Sehschärfe des Patienten eingestellt werden, so dass auf das Tragen zusätzlicher Hilfsmittel wie Brille oder Kontaktlinsen verzichtet werden kann.

Erfindungsgemäß wird die Aufgabe durch die Merkmale des unabhängigen Anspruchs gelöst. Bevorzugte Weiterbildungen und Ausgestaltungen sind Gegenstand der abhängigen Ansprüche.

Die Anordnung zur Beleuchtung/Bestrahlung eines menschlichen Auges, insbesondere der in das Auge implantierten photosensitiven optisch wirksamen Kunststoffe, besteht aus einer Beleuchtungseinheit, einem optischen Abbildungssystem, einer Auswerteeinheit, einer zentralen Steuereinheit und einer Ausgabeeinheit, wobei die Beleuchtungseinheit eine zeitlich und/oder örtlich variable Beleuchtung erzeugt.

Dabei besteht die Beleuchtungseinheit aus einer Beleuchtungsquelle und einem opto-elektronischen Bauelement.

Bei der Anordnung kann als opto-elektronisches Bauelement, welches bezüglich Lichtdurchlässigkeit, Lichtreflexion oder Lichtemission steuerbar ist, ein Mikrodisplay oder ein Mikroscannerspiegel zum Einsatz kommen.

Die zentrale Steuereinheit dient der Eingabe, Erfassung, Verarbeitung und Speicherung von Daten und kann über eine Benutzeroberfläche und ein Interface verfügen.

Weiter kann die Auswerteeinheit aus einer Bildaufnahme- und einer Verarbeitungseinheit bestehen.

Bei der Anordnung kann als Ausgabeeinheit zur Visualisierung und Ausgabe der Daten ein Monitor, ein Drucker und/oder ein HMD (head mounted display) zum Einsatz kommen.

Darüber hinaus kann das optische Abbildungssystem über eine einstellbare numerische Apertur und/oder eine variable Schnitt- oder Brennweite, zur scharfen Abbildung des Beleuchtungsmusters in unterschiedlichen Ebenen verfügen.

Anstelle der Beleuchtungseinheit kann auch ein selbstleuchtendes Array verwendet werden.

Bei der Anordung kann eine Eye-Tracker-Einheit, bestehend aus einer Kamera und einer beispielsweise durch einen Strahlteiler eingekoppelten vorzugsweisen IR-Beleuchtung vorhanden sein.

Bei der Anordnung kann zusätzlich eine Fixiermarke auf das zu behandelnde oder auch das andere, nicht zu behandelnde Auge projiziert werden, die als blinkende und optisch aus dem Unendlichen kommende und/oder auf den Refraktionszustand des Patienten einstellbare Leuchtmarke ausgebildet ist.

Auch ist es möglich, daß bei der Anordnung zusätzlich eine Fixiermarke auf das das zu behandelnde oder auch das andere, nicht zu behandelnde Auge projiziert wird, die als blinkende und optisch aus dem Unendlichen kommende Leuchtmarke angeboten wird und in ihrer Lage und Position frei einstellbar ist.

Die Anordnung kann zur Beleuchtung von bereits in das Auge implantierten photosensitiven Kunststoffen, insbesondere interkornealer Ringe, genutzt werden.

Weiter kann bei dem Gerät zur Beleuchtung/Bestrahlung eines menschlichen Auges die, aus der Beleuchtungsquelle und dem opto-elektronischen Bauelement bestehende Bestrahlungseinheit, als eine eigenständige Einheit ausgebildet sein, die als Zusatzeinheit für verschiedene ophthalmologische Geräte, wie Spaltlampen, Funduskameras, Laserscanner und OPMI-Geräte verwendet werden kann, um Beleuchtungs- bzw. Bestrahlungsstrukturen mit einer definierten Dosis zu erzeugen.

Auch kann bei dem Gerät zur Beleuchtung/Bestrahlung die aus der Beleuchtungsquelle und dem opto-elektronischen Bauelement bestehende Bestrahlungseinheit als eine eigenständige Einheit ausgebildet sein, die als Zusatzeinheit für verschiedene dermatologische Bestrahlungsgeräte verwendet werden kann, um Beleuchtungs- bzw. Bestrahlungsstrukturen mit einer definierten Dosis zu erzeugen.

Bei einem Verfahren zur Beleuchtung/Bestrahlung eines menschlichen Auges, insbesondere beim Betreiben der Anordnung zur Beleuchtung/Bestrahlung eines menschlichen Auges bestehend aus einer Beleuchtungseinheit, einem optischen Abbildungssystem, einer Auswerteeinheit, einer zentralen Steuereinheit und einer Ausgabeeinheit, kann die Beleuchtungseinheit eine zeitlich und/oder örtlich variable Beleuchtung erzeugen.

Bei dem Verfahren kann die Eingabe der zuvor ermittelten Befunddaten per Hand oder durch Übertragung der Daten über das vorhandene Interface oder durch Bestimmung mittels der Anordnung selbst erfolgen.

Bei dem Verfahren kann das von der Beleuchtungseinheit, bestehend aus einer Beleuchtungsquelle und dem opto-elektronischen Bauelement, erzeugte Bestrahlungsmuster von dem Abbildungssystem in eine frei einstellbare Objektebene abgebildet werden.

Bei dem Verfahren kann anhand der von der Auswerteeinheit ermittelten Messwerte eine automatisierte Bildauswertung und/oder Online-Steuerung ermöglicht werden.

Weiter können bei dem Verfahren die ermittelten Daten zur Registrierung, Dokumentation und Auswertung gespeichert werden.

Auch können bei dem Verfahren die Auswertungsergebnisse durch die

Ausgabeeinheit dokumentiert werden.

Bei dem Verfahren können anhand der ermittelten Daten anwendungsspezifischen statische bzw. dynamische Bestrahlungsmuster, zur gezielten räumlichen und zeitlichen Abfolge erzeugt werden.

Bei dem Verfahren können anhand dieser Daten von der zentralen Steuereinheit die für den jeweiligen Einsatzzweck erforderlichen Parameter ermittelt und an die Beleuchtungsquelle weitergeleitet werden.

Weiter kann bei dem Verfahren durch die Eye-Tracker-Einheit kontrolliert werden, ob die erzeugten Beleuchtungsmuster während der Bestrahlung exakt auf die zu bestrahlenden Bereiche des Auges bzw. des photosensitiven Kunststoffes treffen.

Auch können bei dem Verfahren die erzeugten Beleuchtungsmuster mit Hilfe einer Eye-Tracker-Einheit und der Beleuchtungseinheit einer möglichen Augenbewegung nachgeführt werden.

Weiter kann zusätzlich eine Fixiermarke auf das das zu behandelnde oder auch das andere, nicht zu behandelnde Auge projiziert werden, die als blinkende und optisch aus dem Unendlichen kommende Leuchtmarke angeboten wird und in ihrer Lage und Position frei einstellbar ist.

Die erzeugten Beleuchtungsmuster können zur Beleuchtung von bereits in das Auge implantierten photosensitiven Kunststoffen, insbesondere interkornealen Ringen genutzt werden.

Die vorgeschlagene technische Lösung besteht im wesentlichen aus der Beleuchtungseinheit und einem optischen Abbildungssystem und kann sowohl als eigenständige Einheit als auch als Zusatzeinheit für verschiedene ophthalmologische Geräte, wie Spaltlampen, Funduskameras, Laserscanner und OPMI-Geräte verwendet werden. Somit ergibt sich eine breite Anwendung, die sich zudem nicht nur auf das Gebiet der Ophthalmologie beschränkt. Die Bestrahlungseinheit kann auch genauso als Zusatzeinheit für verschiedene dermatologische Bestrahlungsgeräte verwendet werden, um durch gezielte Bestrahlung mit einer variablen Beleuchtung spezifische Wirkung hervorzurufen.

Die Erfindung wird nachfolgend anhand von Ausführungsbeispielen beschrieben. Dazu zeigen:
- Figur 1:: einen möglichen Prinzipaufbau der erfindungsgemäßen Anordnung mit einem Mikrodisplay vom DMD-Typ,
- Figur 2:: einen weiteren möglichen Prinzipaufbau der erfindungsgemäßen Anordnung mit einem Mikrodisplay vom LCOS-Typ und
- Figur 3:: mögliche Beleuchtungsmuster mit der dazugehörigen Intensitätsverteilung.

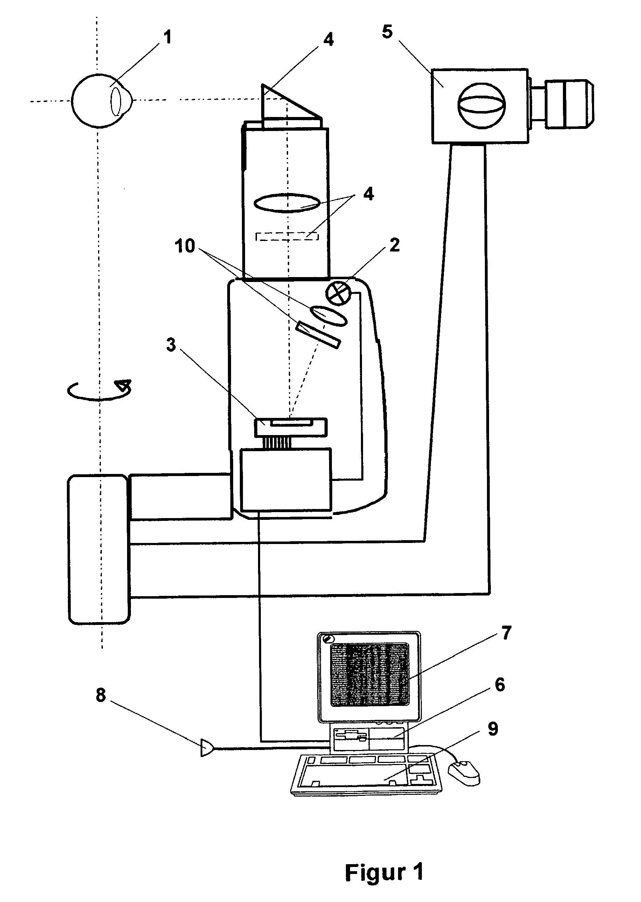

**Figur 1** zeigt den Prinzipaufbau der erfindungsgemäßen Anordnung zur Erzeugung einer zeitlich und/oder örtlich variablen Beleuchtung für die Diagnose und Therapie, insbesondere am menschlichen Auge **1**.

Die Anordnung besteht im wesentlichen aus einer Beleuchtungseinheit, einem optischen Abbildungssystem **4**, einer Auswerteeinheit, einer zentralen

Steuereinheit **6** und einer Ausgabeeinheit **7**, wobei die Beleuchtungseinheit aus einer Beleuchtungsquelle **2** und einem opto-elektronischen Bauelement **3** besteht. Die Beleuchtungsquelle **2** ist hinsichtlich ihrer Intensität und Dauer steuerbar und verfügt dazu über zusätzliche Mittel für die Steuerung und Überwachung des emittierten Lichtes. Als Beleuchtungsquelle **2** dient eine, hinsichtlich der spektralen Zusammensetzung des Lichtbündels, steuerbare Lampe oder Laserquelle. Die Steuerung der spektralen Zusammensetzung kann hierbei über ein Filterrad (nicht dargestellt) erfolgen. Als opto-elektronisches Bauelement **3** wird hierbei ein Mikrodisplay vom DMD-Typ (digital micromirror device) verwendet. Zur Gewährleistung einer gleichmäßigen Ausleuchtung wird für die Übertragung des Lichtstrahles von der Beleuchtungsquelle zum Mikrodisplay eine Lichtleitfaser, ein Glasmischstab, ein Integratorstab oder eine geeignete Kondensoranordnung **10** verwendet. Das vorhandene optische Abbildungssystem **4** verfügt über eine einstellbare numerische Apertur und eine variable Schnittweite zur scharfen Abbildung des Beleuchtungsmusters in unterschiedliche Ebenen des Objektraumes. Damit können unterschiedliche Beleuchtungsmuster entlang der optischen Achse in unterschiedlichen Ebenen des Objektes, z. B. auf der Linsenvorderfläche bzw. Linsenrückfläche erzeugt und somit geometrisch-räumliche Effekte erzielt werden. Diese Möglichkeit der Abbildung der Beleuchtungsmuster in unterschiedlichen Ebenen kann vorteilhafter Weise mit einer Abstandskontrolle und/oder einer Fokussierhilfe zum Auge kombiniert werden. Damit kann die Position auch entlang der optischen Achse genau eingestellt und konstant gehalten werden. Die Fokussierhilfe kann auf dem Prinzip der Mehrfach-Spotabbildung unter hoher Apertur erfolgen, bei der alle einzelnen Spots nur in der Zielebene zusammenfallen und einen einzelnen Spot ergeben. Eine Abstandskontrolle kann dabei beispielsweise über bekannte Vierquadrantenempfänger erfolgen die den Scheitelreflex der Hornhaut auswerten. Mit der variabel einstellbaren numerischen Apertur kann einerseits die Intensität des Beleuchtungsmusters in der Abbildungsebene und andererseits durch die Beeinflussung der Strahldichte an der Retina die Einhaltung der gültigen Grenzwerte für die Beleuchtung im Auge geregelt werden.

Durch Kombination der verstellbaren Aperturblende mit der Funktion der verstellbaren Schnitt- oder Brennweite und der Realisierung dynamischer Beleuchtungsmuster können gezielt Bestrahlungsfolgen mit speziellen Mustern an bestimmten Orten, bei ständiger Kontrolle der zulässigen Strahlendosis, realisiert werden. Eine Positionskontrolle und -korrektur erfolgt mittels einer Eye-Tracker-Einheit und sichert eine exakte Beleuchtung nur im ausgerichteten fokussierten Zustand auch bei Augenbewegungen, so dass auf mechanische Fixierungen des Patientenauges verzichtet werden kann.

Zur Messung, Auswertung, Dokumentation und Ausgabe sind weiterhin ein Beobachtungssystem **5** und eine Auswerteeinheit vorgesehen. Die Auswerteinheit besteht dabei aus einer Bildaufnahme- und einer Verarbeitungseinheit. Die zentrale Steuereinheit **6** zur Eingabe, Erfassung, Verarbeitung und Speicherung von Daten verfügt über eine Benutzeroberfläche **9** und ein Interface **8**. Als Ausgabeeinheit **7** zur Visualisierung und Ausgabe von Daten dient beispielsweise ein Monitor, ein Drucker und/oder ein HMD (head mounted display).

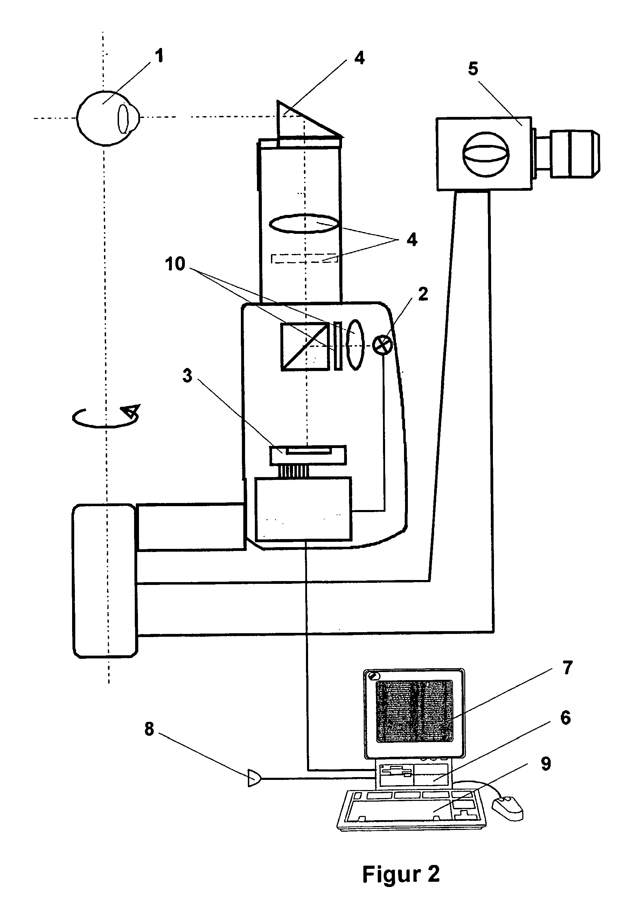

Im Gegensatz dazu zeigt **Figur 2** den Prinzipaufbau der erfindungsgemäßen Anordnung zur Erzeugung einer strukturierten Beleuchtung, bei dem einem Mikrodisplay vom DMD-Typ ein reflektierendes Mikrodisplay vom LCOS-Typ (liquid crystal on silicon) als opto-elektronisches Bauelement **3** zum Einsatz kommt. Es sind aber auch opto-elektronischen Bauelemente **3** vom transmissiven LCD-Typ (liquid crystal display), selbstleuchtenden LED-Typ (light emitting diode) oder OLED-Typ (organic light emitting diode) einsetzbar. Bei dem in **Figur 1** dargestellten Prinzipaufbau könnte statt dem Mikrodisplay auch ein Mikroscannerspiegel mit zwei einzeln ansteuerbaren Schwingungsebenen zum Einsatz kommen.

Bei einem Verfahren zur Erzeugung einer zeitlich und/oder örtlich variablen Beleuchtung, insbesondere beim Betreiben einer der zuvor beschriebenen Anordnungen werden nach der Eingabe der im Vorfeld ermittelten Befunddaten (Refraktionszustand) von der zentralen Steuereinheit **7** die für den Einsatzzweck erforderlichen Parameter für die Beleuchtungsstrahlung ermittelt und an die Beleuchtungsquelle **2** und das opto-elektronische Bauelement **3** weitergeleitet. Anhand der ermittelten Daten können anwendungsspezifische statische aber auch dynamische Bestrahlungsmuster, zur gezielten räumlichen und zeitlichen Abfolge erzeugt werden.

Das Verfahren ist besonders zur Beleuchtung/Bestrahlung von bereits in das Auge implantierten optischen Linsen oder anderen Elementen geeignet. Diese bestehen dabei aus photosensitiven Grundmaterialien, gemäß den Patentschriften WO 00/41650 und WO 01/71411, so dass ihre optischmechanischen Eigenschaften innerhalb einer gewissen Zeit durch die Stimulation mit Licht geändert werden können.

Die zu beleuchtenden Kunststofflinsen können dabei neben Intraokularlinsen (IOL), insbesondere auch Vorderkammerlinsen (z. B. Artisan- und Nuvita-Linsen) oder intraokularen Kontaktlinsen (ICL) sein.

Es gibt aber auch andere optische Elemente, wie beispielsweise sogenannte interkorneale Ringe, die gezielt in der Kornea plaziert werden und durch ihre Form und Lage eine Veränderung der Brechungsverhältnisse der Kornea bewirken. Die Implantation ist für den Patienten relativ schonend, da bis auf eine periphere Eintrittsstelle (Montageöffnung) weder Epithel noch Endothel beschädigt oder verändert werden. Diese interkornealen Ringe werden gezielt in der Kornea plaziert und bewirken durch ihre Form und Lage eine mehr oder weniger gespannten Oberfläche der Kornea und verändern dadurch die Brechungsverhältnisse der Kornea. Da das Verfahren nicht zwangsläufig an die Ringstruktur gebunden ist, sind auch andere Formelemente, wie beispielsweise dünne Scheiben denkbar.

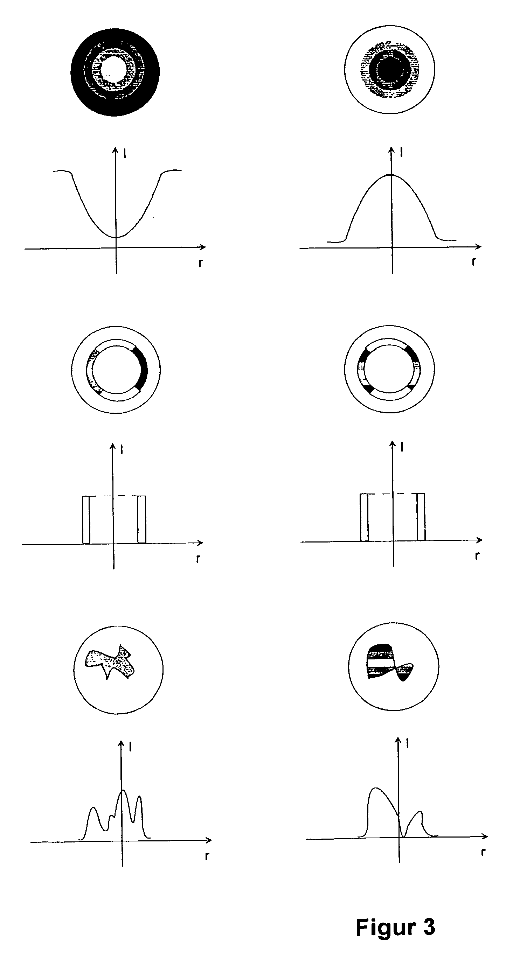

**Figur 3** zeigt mögliche Bestrahlungsmuster und die dazugehörigen Intensitätsverteilungen. Befunddaten können aus Voruntersuchungen mit entsprechenden Messgeräten stammen oder innerhalb des Gerätes selbst bestimmt werden. Hierbei sind als Befunddaten sowohl Ergebnisse einer Wellenfrontanalyse als auch die Daten einer Topographieuntersuchung möglich. Selbst die Kombination der Daten aus verschiedenen Untersuchungen ist sinnvoll und denkbar. Die Übergabe der Befunddaten kann dann durch Eingabe über die Benutzeroberfläche **9** per Hand oder komfortabler durch Übertragung der Daten über das vorhandene Interface **8** erfolgen. Das von der Beleuchtungsquelle **2** und dem opto-elektronischen Bauelement **3** erzeugte Bestrahlungsmuster wird vom Abbildungssystem **4** in eine innerhalb gewisser Grenzen frei einstellbare Ebene im Objektraum abgebildet. Ein vorhandenes Beobachtungssystem **5** dient der visuellen Kontrolle und Beobachtung des Auges **1** während des Bestrahlungsvorganges. Zur automatisierten Bildauswertung und um eine Online-Steuerung zu ermöglichen, werden von einer Bildaufnahme- und Verarbeitungseinheit die entsprechenden Messwerte an die Auswerteeinheit geliefert. Die aufgenommenen Bilder und Daten werden zur Registrierung, Verarbeitung, Dokumentation und Auswertung von der zentralen Steuereinheit **6** weiterverarbeitet, protokolliert und gespeichert. Eine

Ausgabeeinheit **7** dokumentiert die Auswertungsergebnisse.

Besonders vorteilhaft ist der Einsatz einer, aus einer Kamera und einer IR-Beleuchtung bestehenden Eye-Tracker-Einheit. Die Eye-Tracker-Einheit kann z. B. durch einen Strahlteiler angekoppelt werden, überwacht mögliche Augenbewegungen und kontrolliert, ob die erzeugten Beleuchtungsmuster exakt auf die zu bestrahlenden Bereiche des Auges bzw. des photosensitiven Kunststoffes treffen. Überschreitet das Beleuchtungsmuster radial bzw. seitlich einen bestimmten vorher festgelegten Toleranzwert für eine ebenfalls vorher festgelegte Zeitdauer, wird die Bestrahlung unterbrochen und erst bei wieder Erreichen des Zielzustandes fortgesetzt. Die Zeitdauer der Bestrahlung wird dabei ausgewertet um die jeweilige Dosis sicherzustellen und mit den gewünschten Vorgabewerten abzugleichen. Die wählbare Toleranz richtet sich nach der erforderlichen Genauigkeit zum Erreichen des Sollzustandes.

Bei radialem bzw. seitlichem Überschreiten vorher festgelegter Toleranzwerte kann das Beleuchtungsmuster der Augenbewegung gezielt nachgeführt werden. Der Vorteil liegt in der Tatsache, dass der Bestrahlungsvorgang nicht unterbrochen werden muß. Zur Ruhigstellung des Patientenauges kann hierbei zusätzlich eine leuchtende Fixiermarke auf das Auge projiziert werden. Vorteilhafter Weise sollte diese Leuchtmarke blinken und dem Patienten optisch aus dem Unendlichen angeboten werden, um ein entspanntes Sehen und Erkennen der Marke zu ermöglichen. Dabei ist es möglich die Fixiermarke auf das zu behandelnde oder auch das andere, nicht zu behandelnde Auge zu projizieren. Die Fixiermarke kann aber auch zum gezielten Positionieren des Patientenauges in bestimmte Richtungen verwendet werden. Dazu kann vorteilhafter Weise ein weiteres Mikrodisplay verwendet werden, bei dem die Position der Marke ohne bewegte Teile opto-elektronisch verschoben werden kann.

Die Nachführung der Beleuchtungsmuster auf dem Auge kann ebenfalls vorteilhafter Weise ohne jegliche bewegte Teile erfolgen, indem die Muster einfach auf dem opto-elektronischen Bauelement entsprechend der skalierten Vorgabe der Eye-Tracker-Einheit zeitnah verschoben wird.

Die Beleuchtung für die Kamera der Eye-Tracker-Einheit sollte in einem anderen Spektralbereich als die Beobachtungs- bzw. Therapiewellenlänge erfolgen. Dadurch sind gegenseitige Beeinflussungen der Strahlengänge ausgeschlossen. Die Verwendung einer Eye-Tracker-Einheit mit IR-Beleuchtung und entsprechender Nachführung des Musters ist insbesondere dann vorteilhaft, wenn zur Refraktionskorrektur höherer Aberrationen besonders fein strukturierte Beleuchtungsmuster verwendet werden sollen.

Zur visuellen Kontrolle ist es möglich Ziel-, Toleranz- und Nachführfeld in der Beobachtungseinheit anzuzeigen. Dies erfolgt durch Okulareinspiegelung oder durch Abbildung in einer zum Zielobjekt konjugierten Ebene, in der sich ein entsprechender Display befindet.

Bei der Bestrahlung z. B. zur Polymerisation von künstlichen Augenlinsen sind Beleuchtungsquellen **2** erforderlich, die einen hohen UV-Anteil aufweisen, wie beispielsweise Quecksilberbogenlampen, Xenonlampen oder UHP-Lampen. Durch diesen hohen UV-Anteil des Lichtes ist jedoch besonderes Augenmerk auf die zulässige Strahlenbelastung entsprechend den geltendenden Vorschriften zu legen und gegebenenfalls Maßnahmen zur gezielten Abschwächung schädlicher Anteile durch beispielsweise geeignete Kantenfilter zu treffen. Die, aus der Beleuchtungsquelle **2** und dem opto-elektronischen Bauelement **3** bestehende Bestrahlungseinheit, die im Beispiel als eine eigenständige Einheit ausgebildet ist, kann als Zusatzeinheit für verschiedene ophthalmologische Geräte, wie Spaltlampen, Funduskameras, Laserscanner und OPMI-Geräte verwendet werden, um Beleuchtungs- bzw.

Bestrahlungsstrukturen mit einer definierten Dosis zu erzeugen. Die Bestrahlungseinheit kann aber auch genauso als Zusatzeinheit oder eigenständiges Gerät für verschiedene dermatologische Bestrahlungsgeräte verwendet werden.

Die erfinderische Lösung ist hauptsächlichst zum nachträglichen Feinabgleich der Brechkraft von bereits im Auge implantierten photosensitiver Kunststoffen vorgesehen. Dies können sowohl optische Linsen als auch andere optische Elemente sein, die gezielt in der Kornea platziert werden und durch ihre Form und Lage eine Veränderung der Brechungsverhältnisse der Kornea bewirken. Aufgrund der möglichen Anpassung an individuelle Befunddaten ergibt sich durch die Realisierbarkeit dynamischer Vorgänge und eine mögliche online-Regelung weitere Anwendungsgebiete. Denkbar ist beispielsweise die kombinierte Ermittlung der Ausgangsdaten, d. h. des Refraktionszustandes in Form von Wellenfrontanalyse und einer Kornea-Topografie. Während der Belichtung kann die Eye-Tracker-Einheit zur Positionsüberwachung und zur Musternachführung auf dem Auge zum Einsatz kommen, um den Prozess der Positionierung bzw. der Nachführung des Beleuchtungsmusters auch während langen Bestrahlungszeiten von einigen Sekunden besser lösen zu können. Dabei ist es sogar möglich den Refraktionszustand in Abhängigkeit vom erreichten Bearbeitungszustand des zu beleuchtenden Objektes während der Behandlung online zu ermitteln.

Durch die gezielte Strahlablenkung innerhalb der Linse oder mittels weiterer optisch wirksamer Formteile könnte das Bildzentrum bzw. bestimmte Stellen der Abbildung auf andere Areale des Empfängers umgelenkt werden. Das ist sinnvoll für Fälle in denen z. B. die Netzhaut des Patienten in bestimmten Gebieten durch Skotome schwer geschädigt ist und diese somit zum Seheindruck nicht mehr beitragen können. Durch eine gezielte individuelle Veränderung der örtlichen Brechkraft innerhalb der Linse kann dadurch die Abbildung auf gesunde Bereiche der Netzhaut verschoben werden.

Weiterhin ist der Einsatz bei der fotodynamischen Therapie möglich. Hierbei kann durch ein neuartiges Laserverfahren durch Bestrahlung eines neu entwickelten Farbstoffes bislang unaufhaltsam fortschreitende Erkrankungen der Makula nunmehr sogar im Sehzentrum gestoppt werden.

Eine Anwendung ist jedoch auch zur Erzielung dermatologischer Wirkungen denkbar, in dem ebenfalls durch Einbringen oder Zugabe von lichtsensiblen Stoffen und anschließende strukturierte Bestrahlung bestimmte Wirkung erzeugt werden.

## Patentansprüche

1. Anordnung zur Beleuchtung/Bestrahlung eines menschlichen Auges, insbesondere von in das Auge implantiertem photosensitivem optisch wirksamem Kunststoff, umfassend eine Beleuchtungseinheit mit einer Beleuchtungsquelle (2) und einem opto-elektronischen Bauelement (3), ein optisches Abbildungssystem (4), eine Auswerteeinheit, eine zentrale Steuereinheit (6) zur Eingabe, Erfassung, Verarbeitung und Speicherung der Daten und eine Ausgabeeinheit (7), wobei die Beleuchtungseinheit eingerichtet ist, eine zeitlich und/oder örtlich variable Beleuchtung zu erzeugen, ,
und wobei zur Gewährleistung einer gleichmäßigen Ausleuchtung für die Übertragung eines Lichtstrahles von der Beleuchtungsquelle (2) eine Lichtleitfaser, ein Glasmischstab, ein Integratorstab oder eine geeignete Kondensoranordnung vorgesehen ist **dadurch gekennzeichnet, daß** das optische Abbildungssystem (4) über eine einstellbare numerische Apertur verfügt.

2. Anordnung zur Beleuchtung/Bestrahlung eines menschlichen Auges nach Anspruch 1, **dadurch gekennzeichnet, daß** als opto-elektronisches Bauelement (3), welches bezüglich Lichtdurchlässigkeit, Lichtreflexion oder Lichtemission steuerbar ist, ein Mikrodisplay oder ein Mikroscannerspiegel zum Einsatz kommt.

3. Anordnung zur Beleuchtung/Bestrahlung eines menschlichen Auges nach mindestens einem der vorgenannten Ansprüche, **dadurch gekennzeichnet, daß** die zentrale Steuereinheit (6) über eine Benutzeroberfläche (9) und ein Interface (8) verfügt.

4. Anordnung zur Beleuchtung/Bestrahlung eines menschlichen Auges nach mindestens einem der vorgenannten Ansprüche, **dadurch gekennzeichnet, daß** die Auswerteeinheit eine Bildaufnahme- und eine Verarbeitungseinheit umfaßt.

5. Anordnung zur Beleuchtung/Bestrahlung eines menschlichen Auges nach mindestens einem der vorgenannten Ansprüche, **dadurch gekennzeichnet, daß** als Ausgabeeinheit (7) zur Visualisierung und Ausgabe der Daten ein Monitor, ein Drucker und/oder ein HMD (head mounted display) zum Einsatz kommen.

6. Anordnung zur Beleuchtung/Bestrahlung eines menschlichen Auges nach mindestens einem der vorgenannten Ansprüche, **dadurch gekennzeichnet, daß** das optische Abbildungssystem (4) über eine variable Schnitt- oder Brennweite, zur scharfen Abbildung des Beleuchtungsmusters in unterschiedlichen Ebenen verfügt.

7. Anordnung zur Beleuchtung/Bestrahlung eines menschlichen Auges nach mindestens einem der vorgenannten Ansprüche, **dadurch gekennzeichnet, daß** anstelle der Beleuchtungseinheit ein selbstleuchtendes Array verwendet wird.

8. Anordnung zur Beleuchtung/Bestrahlung eines menschlichen Auges nach mindestens einem der vorgenannten Ansprüche, **dadurch gekennzeichnet, daß** eine Eye-Tracker-Einheit, bestehend aus einer Kamera und einer beispielsweise durch einen Strahlteiler eingekoppelten vorzugsweisen IR-Beleuchtung, vorhanden ist.

9. Anordnung zur Beleuchtung/Bestrahlung eines menschlichen Auges nach mindestens einem der vorgenannten Ansprüche, **dadurch gekennzeichnet, daß** zusätzlich eine Fixiermarke auf das zu behandelnde oder auch das andere, nicht zu behandelnde Auge projizierbar ist, die als blinkende und optisch aus dem Unendlichen kommende und/oder auf den Refraktionszustand des Patienten einstellbare Leuchtmarke ausgebildet ist.

10. Anordnung zur Beleuchtung/Bestrahlung eines menschlichen Auges nach mindestens einem der vorgenannten Ansprüche, **dadurch gekennzeichnet, daß** zusätzlich eine Fixiermarke auf das zu behandelnde oder auch das andere, nicht zu behandelnde Auge projizierbar ist, die als blinkende und optisch aus dem Unendlichen kommende Leuchtmarke angeboten wird und in ihrer Lage und Position frei einstellbar ist.

11. Anordnung zur Beleuchtung/Bestrahlung eines menschlichen Auges nach mindestens einem der vorgenannten Ansprüche, **dadurch gekennzeichnet, daß** die Beleuchtungseinheit als eine eigenständige Einheit ausgebildet ist, die als Zusatzeinheit für verschiedene ophthalmologische Geräte, wie Spaltlampen, Funduskameras, Laserscanner und OPMI-Geräte verwendbar ist, um Beleuchtungs- bzw. Bestrahlungsstrukturen mit einer definierten Dosis zu erzeugen.

12. Anordnung zur Beleuchtung/Bestrahlung nach mindestens einem der vorgenannten Ansprüche, **dadurch gekennzeichnet, daß** die, Beleuchtungseinheit als eine eigenständige Einheit ausgebildet ist, die als Zusatzeinheit für verschiedene dermatologische Bestrahlungsgeräte verwendbar ist, um Beleuchtungs- bzw. Bestrahlungsstrukturen mit einer definierten Dosis zu erzeugen.

## Claims

1. Assembly for illumination/irradiation of a human eye, in particular of a photo-sensitive optical plastic material implanted into the eye, said assembly comprising an illumination unit including a source (2) of illumination and an opto-electronic component (3), an optical imaging system (4), an evaluating unit, a central control unit (6) for input, acquisition, processing and storage of data, and an output unit (7), wherein the illumination unit is adapted for generating an illumination that is variable in time and/or space, and wherein an optical fibre, a glass mixing rod, an integrator rod or a suitable condenser assembly is provided so as to ensure uniform illumination for transmission of a light beam from the source (2) of illumination, **characterized in that** the optical imaging system (4) has an adjustable numerical aperture.

2. Assembly for illumination/irradiation of a human eye according to claim 1, **characterized in that** a microdisplay or a microscanner mirror is employed as the opto-electronic component (3), which is controllable with respect to light transmission, light reflection or light emission.

3. Assembly for illumination/irradiation of a human eye according to at least one of the preceding claims, **characterized in that** the central control unit (6) has a user interface (9) and an interface (8).

4. Assembly for illumination/irradiation of a human eye, **characterized in that** the evaluating unit comprises an image-recording unit and a processing unit.

5. Assembly for illumination/irradiation of a human eye according to at least one of the preceding claims, **characterized in that** a screen, a printer and/or an HMD (head mounted display) is/are used as output unit (7) for the display and output of data.

6. Assembly for illumination/irradiation of a human eye according to at least one of the preceding claims, **characterized in that** the optical imaging system (4) has a variable back focal length or focal length for sharp imaging of the illumination pattern in different planes.

7. Assembly for illumination/irradiation of a human eye according to at least one of the preceding claims, **characterized in that** a self-luminous array is used for the illumination unit.

8. Assembly for illumination/irradiation of a human eye according to at least one of the preceding claims, **characterized by** an eye-tracker unit, which consists of a camera and preferably IR illumination coupled in, for example, by a beam splitter.

9. Assembly for illumination/irradiation of a human eye according to at least one of the preceding claims, **characterized in that** additionally a fixation mark is projectable onto the eye that is to be treated or onto the other eye that is not to be treated, said mark being provided as a blinking luminous mark, whose optical origin is at infinity, and/or being adjustable to the patient's refraction condition.

10. Assembly for illumination/irradiation of a human eye according to at least one of the preceding claims, **characterized in that** additionally a fixation mark is projectable onto the eye that is to be treated or onto the other eye that is not to be treated, said mark being provided as a blinking luminous mark and being freely adjustable in terms of its location and position.

11. Assembly for illumination/irradiation of a human eye according to at least one of the preceding claims, **characterized in that** the illumination unit is provided as an independent unit, which can be used as an auxiliary unit for different ophthalmic devices, such as slit lamps, fundus cameras, laser scanners, and OPMI devices, in order to generate illumination or irradiation structures, respectively, at a defined dose.

12. Assembly for illumination/irradiation of a human eye according to at least one of the preceding claims, **characterized in that** the illumination unit is provided as an independent unit, which can be used as an auxiliary unit for different dermatological irradiation devices in order to generate illumination or irradiation structures, respectively, at a defined dose.

## Revendications

1. Agencement pour l'éclairage/l'irradiation d'un oeil humain, en particulier de matière synthétique implantée dans l'oeil, photosensible et efficace du point de vue optique, comprenant une unité d'éclairage avec une source d'éclairage (2) et un composant (3) optoélectronique, un système de reproduction (4) optique, une unité d'analyse, une unité de commande (6) centrale pour l'entrée, l'enregistrement, le traitement et le stockage des données et une unité de sortie (7), l'unité d'éclairage étant aménagée pour générer un éclairage variable dans le temps et/ou localement et une fibre optique, une barre mixte en verre, une barre d'intégrateur ou un dispositif condenseur approprié étant prévus pour garantir un éclairage uniforme pour la transmission d'un faisceau lumineux par la source d'éclairage (2), **caractérisé en ce que** le système de reproduction (4) optique dispose d'une ouverture numérique réglable.

2. Agencement pour l'éclairage/l'irradiation d'un oeil humain selon la revendication 1, **caractérisé en ce qu'**un microaffichage ou un miroir à microscanner est utilisé comme composant (3) optoélectronique, qui peut être contrôlé en ce qui concerne la perméabilité à la lumière, la réflexion lumineuse ou l'émission de lumière.

3. Agencement pour l'éclairage/l'irradiation d'un oeil humain selon au moins l'une quelconque des revendications précédentes, **caractérisé en ce que** l'unité de commande (6) centrale dispose d'une interface utilisateur (9) et d'une interface (8).

4. Agencement pour l'éclairage/l'irradiation d'un oeil humain selon au moins l'une quelconque des revendications précédentes, **caractérisé en ce que** l'unité d'analyse comprend une unité d'enregistrement d'image et une unité de traitement.

5. Agencement pour l'éclairage/l'irradiation d'un oeil humain selon au moins l'une quelconque des revendications précédentes, **caractérisé en ce qu'**un moniteur, une imprimante et/ou un HMD (head mounted display) sont utilisés comme unité de sortie (7) pour la visualisation et la sortie des données.

6. Agencement pour l'éclairage/l'irradiation d'un oeil humain selon au moins l'une quelconque des revendications précédentes, **caractérisé en ce que** le système de reproduction (4) optique dispose d'une focale frontale ou d'une distance focale variable pour la reproduction nette du modèle d'éclairage dans différents plans.

7. Agencement pour l'éclairage/l'irradiation d'un oeil humain selon au moins l'une quelconque des revendications précédentes, **caractérisé en ce qu'**un dispositif autoéclairant est utilisé à la place de l'unité d'éclairage.

8. Agencement pour l'éclairage/l'irradiation d'un oeil humain selon au moins l'une quelconque des revendications précédentes, **caractérisé en ce qu'**une unité oculomètre (Eye-Tracker) constituée d'une caméra et d'un éclairage de préférence infrarouge injecté par exemple par un diviseur de faisceau, est présente.

9. Agencement pour l'éclairage/l'irradiation d'un oeil humain selon au moins l'une quelconque des revendications précédentes, **caractérisé en ce que** en supplément un repère de fixation peut être projeté sur l'oeil à traiter ou également l'autre oeil qui n'est pas à traiter, qui est conçu comme un repère lumineux clignotant et venant visuellement de l'infini et/ ou est conçu comme repère lumineux réglable sur l'état de réfraction du patient.

10. Agencement pour l'éclairage/l'irradiation d'un oeil humain selon au moins l'une quelconque des revendications précédentes, **caractérisé en ce que** en supplément un repère de fixation peut être projeté sur l'oeil à traiter ou également l'autre oeil qui n'est pas à traiter, qui est proposé comme repère lumineux clignotant et venant optiquement de l'infini et est réglable librement dans son emplacement et sa position.

11. Agencement pour l'éclairage/l'irradiation d'un oeil humain selon au moins l'une quelconque des revendications précédentes, **caractérisé en ce que** lorsque l'unité d'éclairage est conçue comme une unité autonome, qui peut être utilisée comme unité supplémentaire pour différents appareils d'ophtalmologie, comme des lampes à fente, des cameras d'ophtalmologie, des scanners laser et des appareils OPMI, afin de générer les structures d'éclairage respectivement d'irradiation avec une dose définie.

12. Agencement pour l'éclairage/l'irradiation d'un oeil humain selon au moins l'une quelconque des revendications précédentes, **caractérisé en ce que** l'unité d'éclairage est conçue comme une unité autonome, qui peut être utilisée comme unité supplémentaire pour différents appareils d'irradiation de dermatologie, afin de générer des structures d'éclairage respectivement d'irradiation avec une dose définie.
